# FASCICULE DE BREVET EUROPEEN

(11) **EP 1 091 747 B1**
(45) Date de publication et mention de la délivrance du brevet: **11.02.2004**
(21) Numéro de dépôt: 99926568.9
(22) Date de dépôt: 29.06.1999
(51) Int. Cl.: A61K 35/08, A61P 29/00, A61P 11/00

(54) **UTILISATION DE SOLUTIONS SALINES ISOOSMOTIQUES POUR LA PREVENTION DES INFLAMMATIONS**
VERWENDUNG VON ISOOSMOTISCHEN SALZLÖSUNGEN ZUR VERHÜTUNG VON ENTZÜNDUNGEN
USE OF ISO-OSMOTIC SALINE SOLUTIONS FOR THE PREVENTION OF INFLAMMATION

(30) Priorité: 29.06.1998 FR 9808250
(43) Date de publication de la demande: 18.04.2001
(73) Titulaire: LABORATOIRES GOEMAR S.A., 35400 Saint-Malo (FR)
(72) Inventeur: YVIN, Jean-Claude, F-35400 Saint-Malo (FR); TABARY, Olivier, F-51100 Reims (FR); JACQUOT, Jacky, F-51100 Reims (FR); PUCHELLE, Edith, F-51100 Reims (FR)
(74) Mandataire: Koch, Gustave
(86) Numéro de dépôt international: PCT/FR1999/001565
(87) Numéro de publication internationale: WO 2000/000209

(56) Documents cités:
- CA-A- 1 313 142
- FR-A- 2 688 133
- HOLMSTROM M ET AL: "Effect of nasal lavage on nasal symptoms and physiology in wood industry workers." RHINOLOGY, (1997 SEP) 35 (3) 108-12, XP002099566
- SEPPEY M ET AL: "Comparative randomised clinical study of tolerability and efficacy of Rhinomer Force 3 versus a reference product in post-operative care of the nasal fossae after endonasal surgery." ORL;JOURNAL OF OTO-RHINO-LARYNGOLOGY AND ITS RELATED SPECIALTIES, (1996 MAR-APR) 58 (2) 87-92, XP002099567
- FRANCOIS M. ET AL: "[Non exteriorisated acute sinusitis in children]. LES SINUSITES AIGUES NON EXTERIORISEES DE L'ENFANT." ANNALES DE PEDIATRIE, (1998) 45/10 (713-718), XP002099568

## Description

L'invention a pour objet un médicament à base de solutions salines isoosmotiques.

Ce médicament est destiné à un traitement pour la prévention et la limitation de la libération des médiateurs chimiques responsables du déclenchement des phénomènes inflammatoires des muqueuses des voies respiratoires humaines, notamment au niveau des fosses nasales et des bronches.

Dans le cas de certaines affections des fosses nasales, celles-ci sont le siège de réactions de défense contre les agressions par les produits souvent irritants que transporte l'air ambiant, ces réactions de défense se traduisant par une accumulation de sécrétions muqueuses et mucopurulentes.

Ces sécrétions ont pour conséquence de conduire à l'obstruction des fosses nasales.

Il est connu par l'article « Effect of nasal lavage on nasal symptoms and physiology in wood industry workers* » publié par M. Holstrôm et al dans « Rhinology », 35, 108-112, 1997 de lutter contre ce type d'obstruction en procédant au lavage des fosses nasales au moyen d'eau de mer isotonique.

Un produit qui donne d'excellents résultats à cet égard est constitué par l'eau de mer isotonique commercialisée sous la marque PHYSIOMER.

Les muqueuses des voies respiratoires sont également le siège d'inflammations; celles-ci peuvent se traduire, soit par un oedème au niveau des muqueuses des fosses nasales qui a son tour provoque un phénomène d'obstruction, rendant impossible la respiration par le nez, soit éventuellement par un oedème des muqueuses bronchiques entraînant des pathologies du type bronchopneumopathies.

Ces inflammations sont le résultat d'une agression de l'organisme, et plus particulièrement des voies respiratoires, par les virus et les bactéries transportés par l'air respiré et également par tous autres éléments irritants et allergisants que transporte cet air, notamment les pollens et tous autres produits qui sont à la base de la pollution atmosphérique.

Il est déjà connu de lutter contre ce type de pathologies en ayant recours à des agents décongestionnants à effet vasocontricteur.

Il est également connu d'utiliser dans ce but des médicaments à base de corticostéroïdes.

Les résultats obtenus avec les agents décongestionnants et avec les corticostéroïdes sont satisfaisants mais ces agents ont des effets secondaires quelquefois gênants.

L'invention a donc pour but surtout de remédier aux inconvénients de l'art antérieur et de fournir pour le traitement des pathologies en question, un médicament exempt d'effets secondaires.

Et la Société Demanderesse --sachant que sous l'action de certains agents pro-inflammatoires d'origine bactérienne, tels que par exemple le lipopolysaccharide de P.Aeruginosa ou LPS, sérotype 10, utilisé à la concentration de 1 µg/ml, les cellules épithéliales, glandulaires, respiratoires humaines libèrent des médiateurs chimiques tels que l'interleukine 8, qui à leur tour déclenchent les phénomènes inflammatoires--, a le mérite d'avoir trouvé, à l'issue de travaux de recherche approfondis, que ce but était atteint en ayant recours à un médicament obtenu par l'utilisation d'une solution saline isoosmotique, notamment d'eau de mer isoosmotique et destiné à un traitement propre à prévenir et à limiter la libération, --sous l'influence des agents pro-inflammatoires comprenant non seulement des substances d'origine bactérienne mais aussi les virus, les bactéries et tous autres éléments irritants et allergisants transportés par l'air respiré-- des médiateurs chimiques responsables du déclenchement des phénomènes inflammatoires des muqueuses des voies respiratoires humaines.

La solution saline isoosmotique dont il vient d'être question est caractérisée :
- par un pH de 7,8 à 8,3
- par une densité de 1,008 à 1,01
- par une teneur en matières sèches de 1 à 2% en poids
- par une osmolarité de 305 à 315 mOs/kg et
- par une constitution chimique résultant, pour ce qui
est de ses principaux éléments, du tableau A ci-après :

**TABLEAU A**

| | |
|---|---|
| Sodium (Na) | de 2000 à 2600 mg/l |
| Potassium (K) | de 40 à 80 mg/l |
| Chlorures (Cl) | de 5800 à 6000 mg/l |
| Calcium (Ca) | de 300 à 400 mg/l |
| Magnésium (Mg) | de 1200 à 1500 mg/l |

En conséquence, l'invention réside dans l'utilisation de solutions salines isoosmotiques, notamment obtenues à partir de l'eau de mer, pour l'obtention d'un médicament destiné à un traitement limitant la libération des médiateurs chimiques responsables du déclenchement des phénomènes inflammatoires des muqueuses des voies respiratoires humaines.

Pour préparer les susdites solutions salines isoosmotiques, on peut procéder comme suit :

On utilise comme matière première une eau de mer prélevée par exemple à une profondeur de 5 à 10 mètres dans une zone à forts mouvements de courant, et caractérisée par une teneur en sels supérieure à 32 g/l.

Cette eau est analysée, décantée puis :
- désodée par une technique d'électrodialyse jusqu'à ce que son isotonicité soit d'environ 9 équivalents-grammes de chlorure de sodium au litre,
- filtrée,
- stockée dans des conditions stériles notamment en cuve en acier inoxydable.

Elle est ensuite analysée une nouvelle fois pour vérifier :
- sa stérilité et
- son isotonicité (physiologique).

Enfin, elle est conditionnée de façon stérile de préférence dans un local spécialement traité sous atmosphère contrôlée.

Les travaux qui sont à la base de l'invention comportent des expériences montrant que les solutions salines isoosmotiques utilisées conformément à l'invention sont moins toxiques que les deux produits de référence bien connus, constituées par le sérum physiologique et le tampon PBS, tout en étant supérieurs à ces deux produits en ce qui concerne leur capacité à prévenir et à limiter la libération sous l'influence des agents pro-inflammatoires des médiateurs chimiques responsables du déclenchement des phénomènes inflammatoires des muqueuses des voies respiratoires.

Les toxicités respectives des solutions salines isoosmotiques utilisées conformément à l'invention, du sérum physiologique et du tampon PBS ont été définies par le biais d'une étude comparative portant sur la viabilité en présence de ces trois produits des cellules respiratoires humaines, c'est-à-dire des cellules que l'on rencontre dans les muqueuses respiratoires.

Pour faire cette étude, on a utilisé des cellules glandulaires respiratoires humaines issues de prélèvements bronchiques humains.

On a ensemencé ces cellules épithéliales respiratoires à la même densité cellulaire dans des plaques de culture de 12 puits.

On les a cultivées pendant 48 heures dans un milieu de culture DMEM/F12 supplémenté d'une part avec 2% d'un nutriment constitué par le produit commercialisé sous la marque ULTROSER G et d'autre part avec une dose d'antibiotiques suffisante pour éviter la contamination bactérienne; on obtient ainsi des cultures en monocouche homogènes et confluentes, soit 310 000 cellules par puits de culture après 48 heures de culture.

Au moment où la confluence est atteinte, les cellules sont lavées avec une solution physiologique (PBS/Ca²⁺ , pH 7.4) et mises en présence d'une part de la solution saline isoosmotique utilisée selon l'invention, d'autre part de sérum physiologique, d'autre part encore de tampon PBS et, enfin, d'un milieu de culture cellulaire de référence constitué par celui connu sous la désignation DMEM/F12.

A l'issue de plusieurs périodes d'incubation, c'est-à-dire de contact cellulaire, à savoir 4 heures, 8 heures, 16 heures et 24 heures, on a évalué la viabilité cellulaire en présence des quatre milieux dont il vient d'être question.

Cette viabilité cellulaire est exprimée par le pourcentage de cellules viables à la fin de chaque période d'incubation dans la solution saline isoosmotique utilisée selon l'invention, dans le sérum physiologique et dans le tampon PBS par rapport au nombre de cellules viables dans le milieu de culture DMEM/F12.

Elle a été évaluée à l'aide du test d'exclusion dit au bleu de Trypan, en solution à 0,4%, suivi d'un comptage avec une cellule de Malassez.

Conformément au test en question, on détache les cellules du support de culture en utilisant de la trypsine; les cellules vivantes sont ensuite comptées dans une solution aqueuse à 0,4% de bleu de Trypan; en effet, le bleu de Trypan est un colorant cellulaire qui ne pénètre pas à l'intérieur d'une cellule vivante, seules les cellules non viables laissant entrer le bleu de Trypan.

Le nombre de cellules vivantes dans le milieu de culture DMEM/F12 étant par définition rapporté à 100, il est possible d'exprimer la viabilité cellulaire dans les autres milieux, en pourcentage par rapport au milieu de culture.

Les résultats de cette étude montrent que les solutions salines isoosmotiques utilisées selon l'invention permettent une meilleure viabilité cellulaire sur des périodes d'incubation longues, c'est-à-dire jusqu'à 24 heures de contact. Le pourcentage de viabilité cellulaire enregistré, à savoir 58%, est supérieur à celui enregistré avec le sérum physiologique, à savoir 48% et a fortiori supérieur à celui enregistré avec le tampon PBS ; ce dernier pourcentage est égal à zéro, la viabilité des cellules dans dans le tampon PBS étant donc nulle.

Dans le tableau B ci-après, on a réuni les pourcentages de viabilité cellulaire enregistrés dans le cadre des expériences qui précédent pour les périodes d'incubation de 4, 8, 16 et 24 heures.

**TABLEAU B**

| **Milieux** | **Pourcentage de viabilité cellulaire** | | | |
|---|---|---|---|---|
| | **Périodes d'incubation** | | | |
| | 4 heures | 8 h | 16 h | 24 h |
| Solution saline isoosmotique | 100 | 85 | 79 | 59 |
| Sérum physiologique | 100 | 81 | 72 | 49 |
| Tampon PBS | 100 | 81 | 66 | 0 |
| Milieu de culture DMEM/F12 | 100 | 100 | 100 | 100 |

La toxicité nettement moindre des solutions salines isoosmotiques par rapport au sérum physiologique et au tampon PBS ressort clairement des valeurs réunies dans le tableau B.

Pour la mise en évidence de la capacité des solutions salines isoosmotiques utilisées conformes à l'invention, à prévenir et à limiter la libération des médiateurs chimiques responsables du déclenchement des phénomènes inflammatoires des muqueuses des voies respiratoires humaines sous l'influence des agents pro-inflammatoires, on incube après stimulation par des agents pro-inflammatoires, les cultures cellulaires concernées dans les solutions salines isoosmotiques utilisées selon l'invention.

Les expériences réalisées par la Société Demanderesse, ont été effectuées sur des cultures sécrétoires glandulaires de la sous-muqueuse bronchique humaine.

Pour ce faire, on cultive les cellules en question dans un milieu de culture, par exemple le milieu de culture RPMI1640, additionné de 2% de milieu ULTROSER G, puis on élimine le milieu de culture, après avoir atteint une confluence de 90% et après avoir introduit l'agent pro-inflammatoire, constitué par le lipopolysaccharide de P. Aeruginosa ou LPS, sérotype 10, utilisé à la concentration de 1 µg/ml.

Après lavage des cellules ainsi traitées, on les réincube :
- dans le même milieu de culture RPMI 1640 seul qui joue le rôle de milieu de contrôle,
- dans le sérum physiologique (solution de NaCl à 0,9% en tant que milieu de référence),
- dans la solution saline isoosmotique utilisée selon l'invention.

La production de médiateur chimique responsable du déclenchement des phénomènes inflammatoires et constitué par l'interleukine-8 par les cellules épithéliales glandulaires sous l'influence de l'agent pro-inflammatoire constitué par le LPS, est estimée par méthode de dosage ELISA (en utilisant le kit de dosage vendu sous la marque MEDGENIX DIAGNOSTIC, Belgique) après une période de temps de contact d'une heure dans les surnageants des trois milieux identifiés ci-dessus.

Les résultats enregistrés pour les trois milieux montrent que la production d'interleukine-8 est :
- de 121 pg/ml/heure dans le cas du milieu RPMI 1640,
- de 65 pg/ml/heure dans le cas du sérum physiologique,
- de 48 pg/ml/heure dans le cas de la solution saline isoosmotique utilisée selon l'invention,
ce qui montre la supériorité de cette dernière du point de vue de sa capacité à prévenir et à limiter la libération sous l'influence des agents pro-inflammatoires des médiateurs chimiques responsables du déclenchement des phénomènes inflammatoires des muqueuses des voies respiratoires.

Ces résultats ont été confirmés par des expériences réalisées sur la culture d'une lignée cellulaire de l'épithelium respiratoire de surface (ligne respiratoire 16-HBE).

Dans la pratique, la solution saline isoosmotique conforme à l'invention est administrée sous forme d'aérosols ou de nébulisats ou par instillation sous forme de gouttes.

Il s'agit d'un médicament destiné à un traitement limitant la libération des médiateurs chimiques responsables du déclenchement des phénomènes inflammatoires des muqueuses des voies respiratoires humaines survenant dans les affections rhinopharingées et pulmonaires.

La solution saline isoosmotique utilisée conformément à l'invention peut être présentée sous la forme d'un Aérosol nasal et buccal à action locale exerçant son effet sur les muqueuses des voies respiratoires humaines, notamment au niveau des fosses nasales et des bronches.

La posologie habituelle peut être de 2 bouffées espacées d'une trentaine de secondes, répétées deux à trois fois par jour en inhalation buccale selon l'état et l'âge du patient.

Dans le cas d'une inhalation nasale, elle est habituellement d'une à deux bouffées dans chaque narine 3 à 4 fois par jour selon l'état et l'âge du patient.

## Revendications

1. Utilisation sous forme d'aérosols ou de nébulisats d'eau de mer rendue isoosmotique, pour l'obtention d'un médicament destiné à un traitement propre à prévenir et à limiter la libération, --sous l'influence des agents pro-inflammatoires comprenant des substances d'origine bactérienne, les virus, les bactéries et tous autres éléments irritants et allergisants transportés par l'air respiré--, des médiateurs chimiques responsables du déclenchement des phénomènes inflammatoires des muqueuses bronchiques et pulmonaires.

2. utilisation selon la revendication 1 d'eau de mer sous forme d'aérosols ou de nébulisats rendue isoosmotique **caractérisée :**
- **par** un pH de 7,8 à 8,3
- par une densité de 1,008 à 1,01
- par une teneur en matières sèches de 1 à 2% en poids
- par une osmolarité de 305 à 315 mOs/kg et
- par une constitution chimique résultant, pour ce qui
est de ses principaux éléments, du tableau A ci-après :
**TABLEAU A**
| | |
|---|---|
| Sodium (Na) | de 2000 à 2600 mg/l |
| Potassium (K) | de 40 à 80 mg/l |
| Chlorures (Cl) | de 5800 à 6000 mg/l |
| Calcium (Ca) | de 300 à 400 mg/l |
| Magnésium (Mg) | de 1200 à 1500 mg/l |

## Claims

1. Use in the form of aerosols or nebulisates of sea water made iso-osmotic, so as to obtain a medication designed for a treatment capable of preventing and limiting the release - under the influence of pro-inflammatory agents comprising substances of bacterial origin, viruses, bacteria or any other irritant or allergenic constituents transported by respired air - of chemical mediators responsible for triggering inflammatory phenomena of the bronchial and pulmonary mucosa.

2. Use according to claim 1 of sea water in the form of aerosols or nebulisates made iso-osmotic, **characterized by**:
- a pH of 7.8 to 8.3
- a density of 1.008 to 1.01
- a dry matter content of 1% to 2 % by weight
- an osmolarity of 305 to 315 mOs/kg
- a chemical constitution resulting, as regards its principal elements, from table A below:
**TABLE A**
| | |
|---|---|
| Sodium (Na) | from 2000 to 2600 mg/l |
| Potassium (K) | from 40 to 80 mg/l |
| Chlorides (Cl) | from 5800 to 6000 mg/l |
| Calcium (Ca) | from 300 to 40 mg/l |
| Magnesium (Mg) | from 1200 to 1500 mg/l |

## Patentansprüche

1. Verwendung isoosmotisch gemachten Meerwassers in Form von Aerosolen oder Zerstäubungen zur Erlangung eines Medikamentes, das für eine Behandlung bestimmt ist, die geeignet ist, - unter dem Einfluss der Pro-Entzündungsagenzien, die Substanzen bakteriellen Ursprungs, Viren, Bakterien und alle anderen reizenden und allergisierenden, durch die Atemluft transportierten Elemente umfassen, - die Freisetzung der chemischen Mediatoren, die für die Auslösung der Entzündungserscheinungen der Bronchial- und Lungenschleimhäute verantwortlich sind, zu verhüten und zu begrenzen.

2. Verwendung von isoosmotisch gemachtem Meerwasser in Form von Aerosolen oder Zerstäubungen nach Anspruch 1, **gekennzeichnet:**
- **durch** einen pH-Wert von 7,8 bis 8,3,
- **durch** eine Dichte von 1,008 bis 1,01,
- **durch** einen Trockensubstanzgehalt von 1 bis 2 % im Gewicht,
- **durch** eine Osmolalität von 305 bis 315 mOs/kg, und
- **durch** eine chemische Zusammensetzung, die sich, was ihre hauptsächlichen Elemente angeht, aus der nachfolgenden Tabelle ergibt:
**TABELLE A**
| | |
|---|---|
| Natrium (Na) | von 2000 bis 2600 mg/l |
| Kalium (K) | von 40 bis 80 mg/l |
| Chloride (Cl) | von 5800 bis 6000 mg/l |
| Kalzium (Ca) | von 300 bis 400 mg/l |
| Magnesium (Mg) | von 1200 bis 1500 mg/l |
